# EUROPÄISCHE PATENTSCHRIFT

(11) **EP 2 311 373 B1**
(45) Veröffentlichungstag und Bekanntmachung des Hinweises auf die Patenterteilung: **01.08.2012**
(21) Anmeldenummer: 09013019.6
(22) Anmeldetag: 15.10.2009
(51) Int. Cl.: A61B 5/151

(54) **Stechsystem zur Entnahme einer Körperflüssigkeit**
Piercing system for removal of a body fluid
Système de piqûre pour l'extraction d'un liquide corporel

(43) Veröffentlichungstag der Anmeldung: 20.04.2011
(73) Patentinhaber: Roche Diagnostics GmbH, 68305 Mannheim (DE); F.Hoffmann-La Roche AG, 4070 Basel (CH)
(72) Erfinder: Winheim, Sven, 6333 Hünenberg (CH); Reinhold, Thomas Sowden, 48161 Münster (DE); Seul, Thomas, 98574 Schmalkalden (DE)
(74) Vertreter: Durm, Frank

(56) Entgegenhaltungen:
- EP-A1- 0 458 451
- EP-A1- 1 254 632
- DE-U1- 8 900 203
- US-A- 4 924 879
- US-A1- 2005 038 465

## Beschreibung

Die Erfindung betrifft ein Stechsystem zur Entnahme einer Körperflüssigkeit aus der Haut eines Menschen oder Tieres. Die Körperflüssigkeit ist in der Regel Blut. In manchen Anwendungsfällen geht es aber auch um die Gewinnung einer Probe von interstitieller Flüssigkeit. Nachfolgend wird ohne Beschränkung der Allgemeinheit auf Blut als Beispiel, auch für andere aus der Haut gewinnbare Körperflüssigkeiten, Bezug genommen.

Zu dem System gehört ein zur einmaligen Verwendung vorgesehenes (disposibles) Stechelement zum Einstechen in die Haut und ein Stechgerät mit einem Antrieb für die Stechbewegung.

Disposible Stechelemente werden seit langem benutzt, um für analytisch-diagnostische Zwecke eine geringe Menge Blut aus einem Körperteil (meist aus einem Finger oder Ohrläppchen) zu entnehmen. In diesem Zusammenhang werden die Stechelemente üblicherweise als Lanzetten bezeichnet. Zum manuellen Einstechen vorgesehene Lanzetten in der Regel nur von medizinisch trainiertem Personal verwendet. Dennoch ist der Einstich mit erheblichem Schmerz verbunden.

Bereits seit langem werden Stechgeräte verwendet, die den Stechantrieb enthalten. Das Stechgerät kann disposibel mit einer fest integrierten Lanzette ausgebildet sein. In der Regel ist es jedoch mehrfach verwendbar und hat ein Kupplungselement, mittels dessen jeweils eine Lanzette auswechselbar mit dem Stechantrieb verbunden werden kann. Da die Geräte und Lanzetten aneinander angepaßte, von dem gleichen Hersteller gelieferte Elemente sind, werden sie als "Stechsystem" oder "Blutentnahmesystem" bezeichnet.

Als Antriebsquelle für den in einem Gehäuse des Stechgerätes angeordneten Stechantrieb dient meist eine Feder. Es werden aber auch andere Antriebsquellen, beispielsweise ein Elektromotor oder ein elektromagnetischer Linearantrieb, verwendet. Durch eine Stechelementführung wird gewährleistet, dass die Stechbewegung auf einem vorbestimmten Einstichweg erfolgt.

Zu Beginn der Entwicklung waren sehr einfache Konstruktionen des Antriebs gebräuchlich, bei denen die Lanzette unmittelbar an einem Ende einer in einem länglichen Gehäuse angeordneten Druckfeder befestigt war (US-Patent 4,469,110). Um die Stechtiefe zu kontrollieren, wird bei solchen Systemen der Bewegungsweg der Lanzette in Einstichrichtung dadurch begrenzt, dass eine Anschlagfläche der Lanzette auf einen korrespondierenden Stechtiefenbegrenzungsanschlag im Gehäuse des Stechgerätes auftrifft.

Bei einer anderen, in dem US-Patent 4,442,836 beschriebenen, Konstruktion wird die Bewegung der Lanzette in Richtung auf die Hautoberfläche bis zu dem Umkehrpunkt (Vortriebsphase der Stechbewegung) von einer ersten Feder angetrieben, während als Antriebsquelle für die Rückzugsbewegung der Lanzette (Rückzugsphase der Stechbewegung) eine zweite Feder dient, die ihre Wirkung entfaltet, nachdem die Kraftkopplung zwischen der ersten Feder und der Lanzette unterbrochen wurde. Dabei soll eine definierte Position des Umkehrpunktes der Lanzettenbewegung dadurch gewährleistet werden, dass die Kraftübertragung zwischen der Antriebsfeder und der Lanzette an einem definierten Punkt des Bewegungsweges unterbrochen wird.

Derartige Blutentnahmesysteme wurden den hohen Anforderungen nicht gerecht, die zu erfüllen sind, wenn eine regelmäßige Überwachung bestimmter analytischer Werte des Blutes erforderlich ist. Dies gilt insbesondere für Diabetiker, die ihren Blutzuckerspiegel häufig kontrollieren sollten, um durch Anpassung von Insulininjektionen an den Bedarf (der abhängig von der Nahrungsaufnahme, der körperlichen Aktivität etc. stark schwankt) ihren Blutzuckerspiegel möglichst ständig innerhalb bestimmter Sollgrenzen zu halten. Durch umfangreiche wissenschaftliche Untersuchungen wurde bewiesen, daß mittels einer Intensivtherapie mit mindestens vier Blutanalysen pro Tag ein dramatischer Rückgang schwerster Spätschäden des Diabetes mellitus (beispielsweise einer Retinopathie mit resultierender Erblindung des Patienten) erreicht werden kann.

Große Fortschritte in dieser Hinsicht wurden durch einen Antriebstyp erreicht, den man als "gehäusegekoppelten Antrieb" bezeichnen kann. Dabei schließt das Getriebe, durch das eine Bewegung der Anriebsquelle (z.B. Feder oder Elektromotor) in eine Stechbewegung des Stechelementes umgewandelt wird, einen Gehäusekopplungsmechanismus ein, der mindestens zwei Getriebeglieder, ein Gehäuselager und ein Kupplungselement zum Ankuppeln des Stechelementes umfasst. Ein erstes gehäuseseitiges Getriebeglied ist mittels des Gehäuselagers an dem Gehäuse des Stechgerätes gelagert und ein zweites stechelementseitiges Getriebeglied ist mit dem Kupplungselement verbunden. Das Stechelement ist über den Gehäusekopplungsmechanismus mit einem Teil des Gehäuses des Stechgerätes derartig verbunden ist, dass es axial in Richtung der Stechbewegung bewegt wird und dabei so mit dem Gehäuseteil verbunden ist, dass es am Umkehrpunkt der Stechbewegung einen vorgegebenen Abstand von dem Gehäuseteil hat. Gehäusegekoppelte Antriebe sind in verschiedenen Ausgestaltungen bekannt:
- Das US-Patent 4,924,879 zeigt ein Stechgerät mit einem an dem Gehäuse gelagerten und von einer Feder angetriebenen Rotor, der über eine Pleuelstange mit dem Stechelement verbunden ist. Dabei bildet der Rotor das gehäuseseitige Getriebeglied und dessen Drehlager das Gehäuselager. Das stechelementseitige Getriebeglied wird von der Pleuelstange gebildet, an die über ein Schwenkgelenk eine Stechelementhalterung angekoppelt ist, die als Kupplungselement zum Ankuppeln des Stechelementes wirkt.
- Das US-Patent 5,318,584 zeigt ebenfalls einen Lanzettenantrieb mit einem Antriebsrotor, auf den einerseits (antriebsseitig) eine Antriebsfeder einwirkt und der andererseits über einer Steuerkurve so mit der Lanzette gekoppelt ist, dass die Rotation des Antriebsrotors in die gewünschte Stechbewegung umgesetzt wird. Auch in diesem Fall bildet der Rotor das gehäuseseitige Getriebeglied und dessen Rotationslager das Gehäuselager. Das stechelementseitige Getriebeglied wird durch einen Steuerkurvenreiter gebildet, der bei der Rotation des Antriebsrotors dessen Steuerkurve abfährt und der fest mit einem Lanzettenhalter verbunden ist, welcher das Kupplungselement zum Ankuppeln der Lanzette bildet.
- Weitere Ausführungsformen gehäusegekoppelter Antriebe mit Rotor werden in dem US-Patent 7,223,276 beschrieben. Auch dort wird das gehäuseseitige Getriebeglied von einem Antriebsrotor mit Steuerkurve und das stechelementseitige Getriebeglied von einem Steuerkurvenreiter gebildet, der fest mit einem Kupplungselement zum Ankuppeln eines Stechelementes verbunden ist. Abweichend von den zuvor erwähnten Ausgestaltungen ist das Kupplungselement in diesem Fall aber kein Stechelementhalter, in dem das Stechelement sitzt und der in dem Gehäuse mit einer Axialführung geführt ist und dadurch das Stechelement indirekt führt. Vielmehr handelt es sich um ein in einer entsprechenden Bohrung des Stechgerätes direkt geführtes Stechelement, an dessen hinterem Ende eine Ausnehmung vorgesehen ist, die das Ankuppeln des Kupplungselementes (in diesem Falle eines Kupplungsstangenkopfes) ermöglicht.
- Das US-Patent 6,858,015 beschreibt eine Konstruktion, bei der das Kupplungselement mittels über Schwenkgelenke gekoppelten Hebeln mit dem Stechgerätgehäuse verbunden ist. Das Kupplungselement wird von einem Lanzettenhalter gebildet, der über ein Schwenkgelenk mit einem gehäuseseitigen Schwenkhebel verbunden ist, welcher das stechelementseitige Getriebeglied bildet. Das gehäuseseitige Getriebeglied wird von dem zweiten Schwenkhebel gebildet, der über ein weiteres Schwenklager an einem Gehäuselager angelenkt ist. Das Gehäuselager ist in diesem Fall nicht unbeweglich an dem Gehäuse befestigt, sondern so ausgebildet, dass es in der Spannphase des Lanzettenantriebs verschiebbar ist. Die Lagerung ist jedoch so gestaltet, dass sich das Gehäuselager in einer präzise definierten Position relativ zu dem Gehäuse befindet, wenn die Stechbewegung abläuft, insbesondere wenn sich das Stechelement an dem für die Präzision der Stechtiefe entscheidenden Umkehrpunkt der Stechbewegung befindet.
- Eine weitere Ausführungsform ist in dem US-Patent 5,196,025 beschrieben.

Die Beispiele zeigen, dass der Begriff "Getriebe" hier im allgemeinen Sinn zu verstehen ist, nämlich als eine kinematische Vorrichtung, die der Kopplung und Umwandlung von Bewegungen dient. Die Getriebeglieder sind die Kopplungselemente eines Getriebes in diesem Sinne. Bei den bekannten gehäusegekoppelten Antrieben wird durch den Gehäusekopplungsmechanismus zumindest am Umkehrpunkt der Stechbewegung eine präzise Kopplung zwischen dem Stechelement und dem Gehäuse derartig bewirkt, dass der Umkehrpunkt der Stechbewegung einen definierten vorgegebenen Abstand zu dem (mindestens am Umkehrpunkt der Stechbewegung gehäusefesten) Gehäuselager hat.

Mittels eines gehäusegekoppelten Antriebs lässt sich eine sehr gute Reproduzierbarkeit der Stechbewegung erreichen, die wiederum Voraussetzung für eine reproduzierbare Stechtiefe und damit für ein geringes Schmerzempfinden bei ausreichender Blutgewinnung ist. Allerdings ist diese Reproduzierbarkeit davon abhängig, dass die Verbindung zwischen dem Gehäuselager und dem Kupplungselement sehr exakt ist. Insbesondere müssen die Drehlager, Schwenklager und Kurvensteuerungen der Getriebeglieder, die die Verbindung herstellen, weitgehend spielfrei und dennoch leichtgängig sein. Dies erfordert einen erheblichen fertigungstechnischen Aufwand, wobei zu berücksichtigen ist, dass die Stechgeräte in großen Stückzahlen zu möglichst günstigen Preisen hergestellt werden sollen.

Der Erfindung liegt auf dieser Grundlage die Aufgabe zugrunde, eine weitere Optimierung der widersprüchlichen Anforderungen hinsichtlich möglichst günstiger Herstellungskosten bei gleichzeitig ausgezeichneter Reproduzierbarkeit zu erreichen.

Die Aufgabe wird durch ein Stechsystem nach Anspruch 1 gelöst.

Die Erfindung kombiniert konstruktive Merkmale zweier bisher als gegenläufig angesehener Konstruktionsprinzipien:
- Einerseits verwendet sie einen gehäusegekoppelten Antrieb, dessen Grundprinzip darin besteht, eine exakt definierte Stichtiefe allein durch die Kopplung zwischen dem Kupplungselement (und damit dem Stechelement) und einem Gehäuselager zu erreichen.
- Andererseits verwendet sie einen bei früheren Geräten mit "frei fliegenden" Stechelementen gebräuchlichen Anschlag.

Um die Kombination dieser scheinbar widersprüchlichen Entwicklungslinien zu ermöglichen, weist der Gehäusekopplungsmechanismus eine Längenausgleichseinrichtung auf, durch die eine Distanzanpassung des Abstandes zwischen dem Stechelement und dem Gehäuselager bei Kontakt der Anschläge und weiterer Bewegung des Stechantriebs gewährleiste wird. Durch diese Distanzanpassung wird verhindert, dass der gehäusegekoppelte Antrieb klemmt und damit funktionsunfähig wird, wenn die beiden Anschläge aufeinander auftreffen. Im Rahmen der Erfindung wurde festgestellt, dass sich dadurch ein optimaler Stechantrieb realisieren lässt, weil einerseits der Gehäusekopplungsmechanismus mit geringerer Präzision und damit kostengünstiger ausgeführt werden kann, andererseits aber durch den Anschlag eine präzise Stichtiefe gewährleistet wird.

Die Erfindung bezieht sich nicht nur auf Stechsysteme, die ausschließlich dazu dienen, einen Blutstropfen für eine anschließende Analyse mit einem anderen Gerät zu gewinnen. Vielmehr richtet sie sich auch auf sogenannte integrierte Systeme, mit denen nicht nur die Blutentnahme, sondern auch die Analyse durchgeführt wird, möglichst ohne zusätzliche Handhabungsschritte durch den Benutzer. Das Stechelement weist dabei meist eine Hohlnadel auf, durch die die Probe angesaugt wird.

Die vorliegende Erfindung richtet sich also generell auf Stechsysteme unterschiedlicher Typen, wobei das Stechelement, das in die Haut eingestochen wird, als massive Nadel (wie bei den erwähnten Lanzetten) oder als Kapillarnadel (mit offener Kapillare oder als geschlossene Hohlnadel) ausgebildet sein kann. Soweit auf Lanzetten oder andere speziellere Ausführungsformen Bezug genommen wird, geschieht dies beispielhaft und ohne Beschränkung der Allgemeinheit. Die erläuterten Sachverhalte gelten grundsätzlich auch für andere Ausgestaltungen.

Die Erfindung wird nachfolgend eines in den Figuren schematisch dargestellten Ausführungsbeispiels erläutert. Die dargestellten Besonderheiten können einzeln oder in Kombination verwendet werden, um bevorzugte Ausgestaltungen der Erfindung zu schaffen.

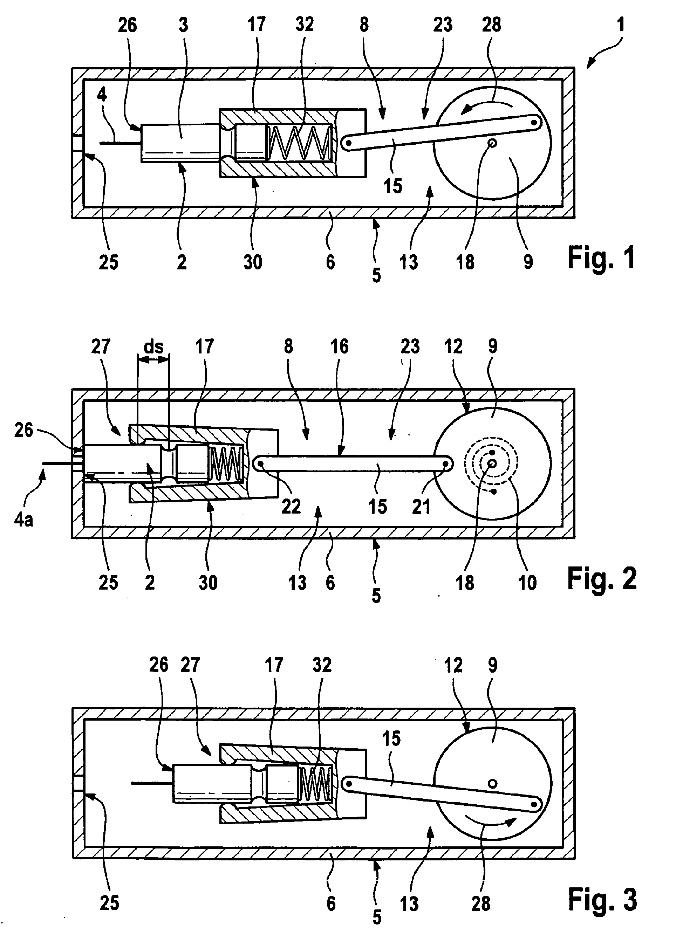

Die Figuren 1 bis 3 zeigen eine schematische Seitenansicht eines erfindungsgemäßen Stechsystems mit geöffnetem Gehäuse in drei verschiedenen Bewegungsphasen.

Das in den Figuren in stark schematisierter Form dargestellte Stechsystem 1 schließt ein Stechelement 2 mit einem Stechelementkörper 3 und einer Nadel 4 sowie ein Stechgerät 5 mit einem Gehäuse 6 ein. In dem Gehäuse 6 befindet sich ein insgesamt mit 8 bezeichneter Stechantrieb.

Zu dem Stechantrieb 8 gehört ein Antriebsrotor 9, der von einer Antriebsquelle 10, hier einer von dem Antriebsrotor 9 verdeckten und deshalb (der Übersichtlichkeit halber nur in Figur 2) gestrichelt dargestellten, Spiralfeder rotierend angetrieben werden kann. Der Antriebsrotor 9 bildet ein erstes, gehäuseseitiges Getriebeglied 12 eines insgesamt mit 13 bezeichneten Getriebes. Zu dem Getriebe 13 gehört außerdem eine Pleuelstange 15, die ein zweites stechelementseitiges Getriebeglied 16 bildet, sowie ein Kupplungselement 17 zum Ankuppeln des Stechelementes 2. Das gehäuseseitige Getriebeglied ist mittels eines Gehäuselagers 18, hier der Rotationswelle des Antriebsrotors 9, an dem Gehäuse 6 des Stechgerätes 5 gelagert. Das zweite Getriebeglied 16, also die Pleuelstange 15, ist jeweils über ein Schwenklager 20, 21 einerseits an dem Antriebsrotor 9 und andererseits an dem Kupplungselement 17 angelenkt.

Die Getriebeglieder 12, 16, das Gehäuselager 18 und das Kupplungselement 17 bilden einen Gehäusekopplungsmechanismus 23, durch den das Stechelement 2 während seiner auf einem vorbestimmten Einstichweg ablaufenden Stechbewegung mit dem Gehäuse gekoppelt ist. Der Stechantrieb 8 ist demzufolge ein gehäusegekoppelter Antrieb. Im Gegensatz zu vorbekannten Stechgeräten ist er aber nicht so ausgebildet, dass das Stechelement 2 am Umkehrpunkt der Stechbewegung einen vorgegebenen Abstand von demjenigen Gehäuseteil hat, an dem sich das Gehäuselager 18 befindet, und dadurch der Umkehrpunkt der Stechbewegung und die Stechtiefe durch den Gehäusekopplungsmechanismus 23 bestimmt wird. Vielmehr ist in dem Gehäuse 6 ein Stechtiefenbegrenzungsanschlag 25 so positioniert, dass eine an dem Stechelement 2 entsprechend angeordnete Anschlagfläche 26 während der Vortriebsphase der Stechbewegung den Stechtiefenbegrenzungsanschlag 25 kontaktiert, so dass die Bewegung des Stechelementes 2 in Einstichrichtung durch die beiden Anschläge 25, 26, d.h. durch Kontakt der Anschlagfläche (26) mit dem Stechtiefenbegrenzungsanschlag (25), begrenzt wird. Bei der dargestellten bevorzugten Ausführungsform befindet sich die Anschlagfläche 26 an der in Einstichrichtung vorderen Stirnseite des Stechelementes 3.

Ein Verklemmen des Antriebs wird dabei durch eine Längenausgleichseinrichtung 27 vermieden, die eine Reduzierung des Abstandes zwischen dem Stechelement 2 und dem Gehäuselager 18 bei Kontakt der Anschlagfläche (26) mit dem Stechtiefenbegrenzungsanschlag (25) und weiterer Bewegung des Stechantriebs 8 gewährleistet. Dies wird nachfolgend anhand der in den Figuren 1 bis 3 dargestellten Bewegungsphasen des Stechantriebs 8 erläutert.

Figur 1 zeigt den Stechantrieb im gespannten Zustand des Rotors 9, bevor die Stechbewegung durch Lösen eines nicht dargestellten Auslösers gestartet wird. Nach dem Auslösen dreht sich der Antriebsrotor 9 in Richtung des Pfeils 28 und die Drehbewegung wird durch die Pleuelstange 15 in eine translatorische Bewegung des Kupplungselementes 17 und damit des Stechelementes 2 auf einem vorbestimmten Einstichweg umgesetzt, der, wie üblich, auf der Längsachse des Stechgerätes 5 verläuft. Dabei wird das im dargestellten Fall als Lanzettenhalter 30 ausgebildete Kupplungselement 17 von einer der Übersichtlichkeit halber nicht dargestellten Führung geführt.

Der Gehäusekopplungsmechanismus 23 des Stechantriebs 8 ist so dimensioniert, dass die Anschlagfläche 26 des Stechelementes 2 auf den Stechtiefenbegrenzungsanschlag 25 des Gehäuses 6 auftrifft, bevor der Umkehrpunkt der Bewegung des Gehäusekopplungsmechanismus 23 erreicht ist. In dem Moment, in dem die beiden Anschläge 25, 26 in Kontakt sind, tritt die Längenausgleichsvorrichtung 27 in Funktion. Bei der dargestellten bevorzugten Ausführungsform wird die Distanzanpassung (d.h. Verminderung) des Abstandes zwischen dem Stechelement 2 und dem Gehäuselager 18 durch eine axiale Verschiebung des Stechelementes 2 relativ zu dem Kupplungselement 17 erreicht. Das Stechelement wird im wesentlichen durch Reibschluss gehalten. Die Haltekraft sollte jedenfalls so hoch sein, dass die Verschiebung erst bei Kontakt der Anschlagfläche (26) mit dem Stechtiefenbegrenzungsanschlag (25) beginnt, andererseits aber auch niedrig genug, dass durch die Reibung die Bewegung des Stechantriebs nicht in einem seine Funktion störenden Ausmaß behindert wird.

Figur 2 zeigt den Endpunkt dieser Verschiebung, der bei maximaler Längenausdehnung des Gehäusekopplungsmechanismus 23 erreicht wird. Dabei steht die Spitze 4a der Nadel 4 aus dem Gehäuse 6 hervor, so dass sie in die Haut eines an der Gehäuseöffnung platzierten Körperteils eindringt. Die durch die Verschiebung bewirkte Distanzanpassung, also im dargestellten Fall die Gesamtverschiebung des Stechelementes 2 relativ zu dem Kupplungselement 17, ist mit ds bezeichnet. Sie ist der Deutlichkeit halber stark übertrieben dargestellt. Vorzugsweise sollte die Distanzanpassung ds nach dem Kontakt der Anschläge bis zum Umkehrpunkt der Stechbewegung maximal 0,5 mm, bevorzugt maximal 0,4 mm und besonders bevorzugt maximal 0,3 mm betragen. Vorzugsweise ist zwischen dem Kupplungselement 17 und dem Stechelement 2 ein Federelement 32 angeordnet, das der für die Distanzanpassung erforderlichen Längsverschiebung entgegenwirkt. Dadurch kann die Präzision der Stichtiefeneinstellung nochmals erhöht werden.

Figur 3 zeigt den Endpunkt der Stechbewegung, bei dem der Antriebsrotor 9 eine nahezu vollständige Drehung absolviert und dabei über die Pleuelstange 15 das Kupplungselement 17 und mit ihm das Stechelement 2 wieder zurückgezogen hat.

Es sind zahlreiche Variationen der Erfindung möglich, die sich insbesondere auf die Ausgestaltung des Lanzettenantriebs und der Distanzanpassung beziehen können. So kann der dargestellte Pleuelantrieb durch einen der anderen einleitend erläuterten Rotorantriebe ersetzt werden, wobei die Rotationsachse des Antriebsrotors sowohl in Richtung der Stechbewegung, als auch quer dazu, verlaufen kann. Auch ein Schwenkhebelantrieb (ähnlich wie eingangs erläutert) kann vorteilhaft eingesetzt werden. Die Längenausgleichseinrichtung kann auch an einer anderen Stelle des Gehäusekopplungsmechanismus vorgesehen sein. Insbesondere kann sie im Bereich des Gehäuselagers derartig realisiert sein, dass nach dem Kontakt der Anschläge eine axiale Verschiebung des Gehäuselagers relativ zu dem Gehäuse möglich ist. Eine geeignete Konstruktion ist (dort zu einem anderen Zweck, nämlich im Zusammenhang mit dem Spannvorgang) in dem eingangs erwähnten US-Patent 6,858,015 beschrieben.

## Patentansprüche

1. Stechsystem (1) zur Entnahme von Körperflüssigkeit aus der Haut eines Menschen oder Tieres, mit
einem Stechelement (2) mit einer Spitze (4a) zum Einstechen in die Haut, und
einem Stechgerät (5), das einen Stechantrieb (8) einschließt, durch den eine Stechbewegung eines Stechelementes (2), das mit dem Stechantrieb (8) verbunden ist, angetrieben wird,
wobei
der Stechantrieb (8) eine Antriebsquelle (10) und ein Getriebe (13) umfasst, durch das eine Bewegung der Antriebsquelle (10) in eine Stechbewegung des Stechelementes (2) umgewandelt wird, bei der das Stechelement (2) in einer Vortriebsphase der Stechbewegung entlang einem vorbestimmten Einstichweg in einer Einstichrichtung bewegt wird, bis die Spitze (4a) des Stechelementes in die Haut eindringt und bei der in einer Rückzugsphase der Stechbewegung das Stechelement (2) nach Erreichen eines der Stechtiefe in die Haut entsprechenden Umkehrpunktes wieder zurückgezogen wird, und
das Getriebe (13) einen Gehäusekopplungsmechanismus (23) mit mindestens zwei Getriebegliedern (12,16), einem Gehäuselager (18) und einem Kupplungselement (17) zum Ankuppeln des Stechelementes (2) einschließt, wobei ein erstes, gehäuseseitiges Getriebeglied (12) mittels des Gehäuselagers (18) an dem Gehäuse (6) des Stechgerätes (5) gelagert ist und ein zweites, stechelementseitiges Getriebeglied (16) mit dem Kupplungselement (17) verbunden ist,
**dadurch gekennzeichnet, dass**
in dem Gehäuse (6) ein Stechtiefenbegrenzungsanschlag (25) so positioniert ist, dass eine Anschlagfläche (26) des Stechelementes (2) während der Vortriebsphase der Stechbewegung den Stechtiefenbegrenzungsanschlag (25) kontaktiert, so dass die Bewegung des Stechelementes (2) in Einstichrichtung durch Kontakt der Anschlagfläche (26) des Stechelementes (2) mit dem Stechtiefenbegrenzungsanschlag (25) begrenzt wird und
der Gehäusekopplungsmechanismus (23) eine Längenausgleichseinrichtung (27) aufweist, um eine Distanzanpassung des Abstandes zwischen dem Stechelement (2) und dem Gehäuselager (18) bei Kontakt der Anschlagfläche (26) des Stechelementes (2) mit dem Stechtiefenbegrenzungsanschlag (25) und weiterer Bewegung des Stechantriebs (8) zu gewährleisten.

2. Stechsystem nach Anspruch 1, **dadurch gekennzeichnet, dass** die Längenausgleichseinrichtung (27) zwischen dem Kupplungselement (17) und dem Stechelement (2) derart ausgebildet ist, dass sie eine axiale Verschiebung des Stechelementes (2) relativ zu dem Kupplungselement (17) nach dem Kontakt der Anschläge erlaubt.

3. Stechsystem nach Anspruch 1, **dadurch gekennzeichnet, dass** die Längenausgleichseinrichtung im Bereich des Gehäuselagers (18) derartig ausgebildet ist, dass sie ein axiale Verschiebung des Gehäuselagers (18) relativ zu dem Gehäuse (6) nach dem Kontakt der Anschläge erlaubt.

4. Stechsystem nach einem der vorhergehenden Ansprüche, **dadurch gekennzeichnet, dass** das Kupplungselement (17) ein Stechelementhalter (30) ist, der in dem Gehäuse (6) mit einer Axialführung gelagert ist.

5. Stechsystem nach einem der vorhergehenden Ansprüche, **dadurch gekennzeichnet, dass** zwischen dem Kupplungselement (17) und dem Stechelement ein Federelement (32) angeordnet ist, das in Einstichrichtung auf das Stechelement wirkt.

6. Stechsystem nach einem der vorhergehenden Ansprüche, **dadurch gekennzeichnet, dass** die Verbindung zwischen dem Kupplungselement (17) und dem Stechelement (2) reibschlüssig ist.

7. Stechsystem nach einem der vorhergehenden Ansprüche, **dadurch gekennzeichnet, dass** der Stechtiefenbegrenzungsanschlag (25) relativ zu der Anschlagfläche (26) des Stechelementes (2) so positioniert ist, dass die Distanzanpassung (ds) nach dem Kontakt der Anschläge (25,26) bis zum Umkehrpunkt der Stechbewegung maximal 0,5 mm, bevorzugt maximal 0,4 mm und besonders bevorzugt maximal 0,3 mm beträgt.

8. Stechsystem nach einem der vorhergehenden Ansprüche, **dadurch gekennzeichnet, dass** das Stechelement (2) eine metallene Nadel (4) und einen Stechelementkörper (3) aus Kunststoff aufweist und sich die Anschlagfläche (26) an einer in Einstichrichtung vorderen Stirnseite des Stechelementkörpers (3) befindet.

## Claims

1. Lancing system (1) for withdrawing body fluid from the skin of a human or animal, the system comprising
a lancing element (2) having a tip (4a) for piercing into the skin, and a lancing instrument (5), including a lancing drive (8) which drives a lancing movement of a lancing element (2), which is connected to a lancing drive (8),
wherein
the lancing drive (8) comprises a drive source (10) and a transmission (13), by which a movement of the drive source (10) is converted into a lancing movement of the lancing element (2), the lancing movement including a forward phase during which the lancing element (2) is moved along a predetermined piercing path in a piercing direction until the tip (4a) of the lancing element penetrates into the skin and a retraction phase during which the lancing element (2) is retracted after reaching a reversal point corresponding to the lancing depth into the skin, and
the transmission (13) including a housing coupling mechanism (23) which comprises at least two transmission links (12, 16), a housing bearing (18), and a coupling element (17) adapted for coupling a lancing element (2) thereto, wherein a first, housing-side transmission link (12) is connected by the housing bearing (18) with the housing (6) of the lancing instrument (5) and a second, lancing-element-side transmission link (16) is connected to the coupling element (17),
**characterized in that**
in the housing (6) a lancing depth limiting stop (25) is so positioned that a stop surface (26) of the lancing element (2) contacts the lancing depth limiting stop (25) during the forward phase of the lancing movement, such that the movement of the lancing element (2) in the piercing direction is limited by contact of the stop surface (26) of the lancing element (2) with the lancing depth limiting stop (25), and
the housing coupling mechanism (23) has a length compensation device (27) to ensure a distance adaptation of the spacing between the lancing element (2) and the housing bearing (18) when the stop surface (26) of the lancing element (2) contacts the lancing depth limiting stop (25) and the lancing drive (8) continues to move.

2. Lancing system according to claim 1, **characterized in that** the length compensation device (27) between the coupling element (17) and the lancing element (2) is adapted to allow an axial displacement of the lancing element (2) relative to the coupling element (17) after the contact of the stops.

3. Lancing system according to claim 1, **characterized in that** the length compensation device is provided in the area of the housing bearing (18) in such a manner that it allows an axial displacement of the housing bearing (18) relative to the housing (6) after the contact of the stops.

4. Lancing system according to any one of the preceding claims, **characterized in that** the coupling element (17) is a lancing element holder (30), which is borne by an axial guide in the housing (6).

5. Lancing system according to any one of the preceding claims, **characterized in that** a spring element (32), which acts in the piercing direction on the lancing element, is positioned between the coupling element (17) and the lancing element.

6. Lancing system according to any one of the preceding claims, **characterized in that** the connection between the coupling element (17) and the lancing element (2) is a friction-type connection.

7. Lancing system according to any one of the preceding claims, **characterized in that** the lancing depth limiting stop (25) is positioned relative to the stop surface (26) of the lancing element (2) so that the distance adaptation (ds) after the contact of the stops (25, 26) up to the reversal point of the lancing movement is at most 0.5 mm, preferably at most 0.4 mm, and particularly preferably at most 0.3 mm.

8. Lancing system according to any one of the preceding claims, **characterized in that** the lancing element (2) has a metal needle (4) and a lancing element body (3) made of plastic, and the stop surface (26) is located on a forward front side of the lancing element body (3) in the piercing direction.

## Revendications

1. Système de piqûre (1) pour le prélèvement de liquide corporel au niveau de la peau d'un homme ou d'un animal, comprenant
un élément de piqûre (2) doté d'une pointe (4a) pour piquer la peau, et
un appareil de piqûre (5) comprenant une commande de piqûre (8) par laquelle est commandé un mouvement de piqûre d'un élément de piqûre (2) relié à la commande de piqûre (8),
la commande de piqûre (8) comprenant une source motrice (10) et une transmission (13) par laquelle un mouvement de la source motrice (10) est transformé en un mouvement de piqûre de l'élément de piqûre (2), où, dans une phase de propulsion du mouvement de piqûre, l'élément de piqûre (2) est mû le long d'une trajectoire de piqûre prédéterminée dans un sens de piqûre jusqu'à ce que la pointe (4a) de l'élément de piqûre pénètre dans la peau, et où dans une phase de retour du mouvement de piqûre, après avoir atteint un point d'inversion correspondant à la profondeur de pénétration dans la peau, l'élément de piqûre (2) est de nouveau mû en arrière, et
la transmission (13) comprenant un mécanisme de couplage de boîtier (23) avec au moins deux organes de transmission (12, 16), un support de boîtier (18) et un élément de couplage (17) pour accoupler l'élément de piqûre (2), un premier organe de transmission (12) côté boîtier étant logé au moyen du support (18) sur le boîtier (6) de l'appareil de piqûre (5) et un second organe de transmission (16) côté élément de piqûre étant relié à l'élément de couplage (17),
**caractérisé en ce que**
une butée (25) limitant la profondeur de piqûre est positionnée de telle façon dans le boîtier (6) qu'une surface de butée (26) de l'élément de piqûre (2) vient en contact avec la butée (25) limitant la profondeur de piqûre pendant la phase de propulsion du mouvement de piqûre, de telle sorte que le mouvement de l'élément de piqûre (2) dans le sens de piqûre est limité par le contact de la surface de butée (26) de l'élément de piqûre (2) avec la butée (25) limitant la profondeur de piqûre, et
le mécanisme de couplage de boîtier (23) comporte un dispositif de compensation de longueur (27), afin de garantir un ajustement de distance de l'écart entre l'élément de piqûre (2) et le support de boîtier (18) en cas de contact de la surface de butée (26) de l'élément de piqûre (2) avec la butée (25) limitant la profondeur de piqûre et de mouvement supplémentaire de la commande de piqûre (8).

2. Système de piqûre selon la revendication 1, **caractérisé en ce que** le dispositif de compensation de longueur (27) entre l'élément de couplage (17) et l'élément de piqûre (2) est conçu de manière à permettre un déplacement axial de l'élément de piqûre (2) par rapport à l'élément de couplage (17) après le contact des butées.

3. Système de piqûre selon la revendication 1, **caractérisé en ce que** le dispositif de compensation de longueur (27) au niveau du support de boîtier (18) est conçu de manière à permettre un déplacement axial du support de boîtier (18) par rapport au boîtier (6) après le contact des butées.

4. Système de piqûre selon l'une des revendications précédentes, **caractérisé en ce que** l'élément de couplage (17) est un support d'élément de piqûre (30), qui est logé dans le boîtier (6) avec un guidage axial.

5. Système de piqûre selon l'une des revendications précédentes, **caractérisé en ce qu'**un élément élastique (32) est disposé entre l'élément de couplage (17) et l'élément de piqûre et agit sur l'élément de piqûre dans le sens de piqûre.

6. Système de piqûre selon l'une des revendications précédentes, **caractérisé en ce que** la liaison entre l'élément de couplage (17) et l'élément de piqûre (2) est une liaison par friction.

7. Système de piqûre selon l'une des revendications précédentes, **caractérisé en ce que** la butée (25) limitant la profondeur de piqûre est positionnée par rapport à la surface de butée (26) de l'élément de piqûre (2) de telle façon que l'ajustement de distance (ds) après le contact des butées (25, 26) jusqu'au point d'inversion du mouvement de piqûre est au maximum de 0,5 mm, de préférence au maximum de 0,4 mm et de manière particulièrement préférée au maximum de 0,3 mm.

8. Système de piqûre selon l'une des revendications précédentes, **caractérisé en ce que** l'élément de piqûre (2) comporte une aiguille métallique (4) et un corps d'élément de piqûre (3) en matière plastique et **en ce que** la surface de butée (26) se trouve vu dans le sens de piqûre, sur une face avant du corps d'élément de piqûre (3).
